# EUROPEAN PATENT APPLICATION

(11) **EP 4 302 938 A1**
(43) Date of publication of application: **10.01.2024**
(21) Application number: 23183145.4
(22) Date of filing: 03.07.2023
(51) Int. Cl.: B25J 11/00, B25J 9/16, A61B 90/20, A61B 90/00, G05B 23/02, G06T 19/00

(54) **DISPLAY SYSTEM AND TEACHING SYSTEM**

(30) Priority: 04.07.2022 JP 2022107569
(71) Applicant: Seiko Epson Corporation, Tokyo 160-8801 (JP)
(72) Inventor: MATSUURA, Yuichiro, Suwa-shi, Nagano, 392-8502 (JP); SEKI, Ryutaro, Suwa-shi, Nagano, 392-8502 (JP); TOGASHI, Daiki, Sakata-shi, Yamagata, 998-0194 (JP)
(74) Representative: MERH-IP Matias Erny Reichl Hoffmann Patentanwälte PartG mbB

(57) **Abstract**

A display system displaying information on a robot arm when the robot arm having a joint is moving, includes a plurality of imaging apparatuses imaging markers provided on the robot arm, a plurality of projection apparatuses projecting the information on the robot arm, and a control apparatus controlling operation of the projection apparatuses to project the information to follow the moving robot arm based on images of the markers captured by the imaging apparatuses.

## Description

The present application is based on, and claims priority from JP Application Serial Number 2022-107569, filed July 4, 2022, the disclosure of which is hereby incorporated by reference herein in its entirety.

### BACKGROUND

### 1. Technical Field

The present disclosure relates to a display system and a teaching system.

### 2. Related Art

JP-A-2021-167065 discloses a machine teaching terminal communicably coupled to a robot and used for teaching of the robot within a work area of the robot. The machine teaching terminal includes a touch panel display receiving input by a worker, a teaching area output unit outputting a teaching area to the touch panel display, an input detection unit detecting the input to the touch panel display, and a teaching unit teaching according to the input.

When teaching the robot, the worker holds the machine teaching terminal in a hand and repeats touch operation on the touch panel display. Thereby, a teaching signal in response to a touched position is transmitted to a control apparatus of the robot and teaching is performed.

When the teaching is completed, the worker reproduces a taught motion and checks a motion of a robot arm. In this regard, it is necessary for the worker to compare the real motion of the robot arm with information output to the touch panel display of the machine teaching terminal. That is, the worker is required to frequently move the point of view between the robot arm and the touch panel display. Accordingly, there is a problem that work efficiency is not sufficiently increased in the work to check the taught motion.

### SUMMARY

A display system according to an application example of the present disclosure is a display system displaying information on a robot arm when the robot arm having a joint is moving, includes a plurality of imaging apparatuses imaging markers provided on the robot arm, a plurality of projection apparatuses projecting the information on the robot arm, and a control apparatus controlling operation of the projection apparatuses to project the information to follow the moving robot arm based on images of the markers captured by the imaging apparatuses.

A teaching system according to an application example of the present disclosure includes the display system according to the application example of the present disclosure, and a teaching apparatus teaching a motion of a robot having the robot arm.

### BRIEF DESCRIPTION OF THE DRAWINGS

FIG. 1 is a perspective view showing an example of a robot system to which a display system and a teaching system according to the present disclosure are applied.
FIG. 2 is a block diagram for explanation of a basic configuration of the robot system shown in FIG. 1.
FIG. 3 is a block diagram showing a configuration and functions of the teaching system according to an embodiment.
FIG. 4 is a perspective view showing a placement example of the teaching system and the robot system shown in FIG. 3.
FIG. 5 is a side view showing examples of markers and canvases attached to an outer surface of a robot.
FIG. 6 is a table showing examples of the markers.
FIG. 7 is a flowchart for explanation of an example of a teaching method using the teaching system according to the embodiment.
FIG. 8 is a table showing examples of types of information displayed by the display system according to the embodiment.

### DESCRIPTION OF EXEMPLARY EMBODIMENTS

As below, a display system and a teaching system according to the present disclosure will be explained in detail based on an embodiment shown in the accompanying drawings.

### 1. Robot

First, an example of a robot system to which the display system and the teaching system according to the present disclosure are applied is explained.

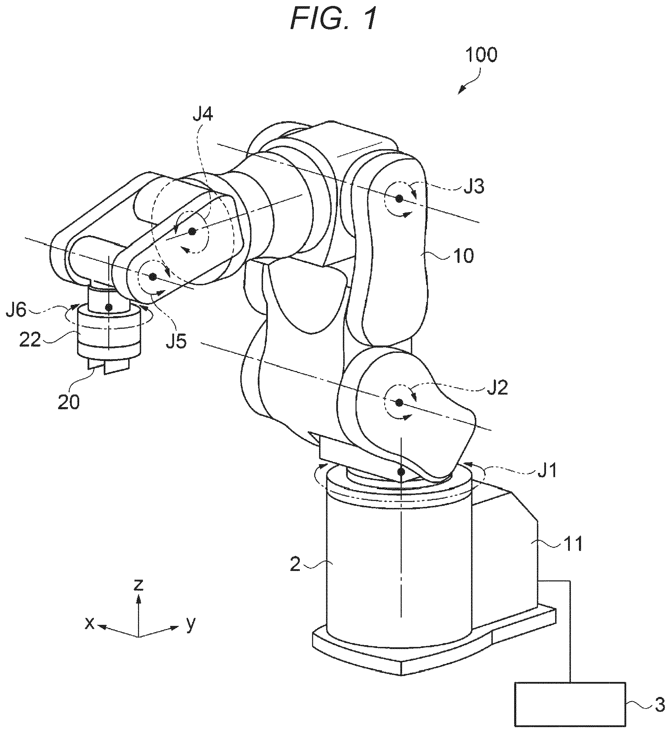

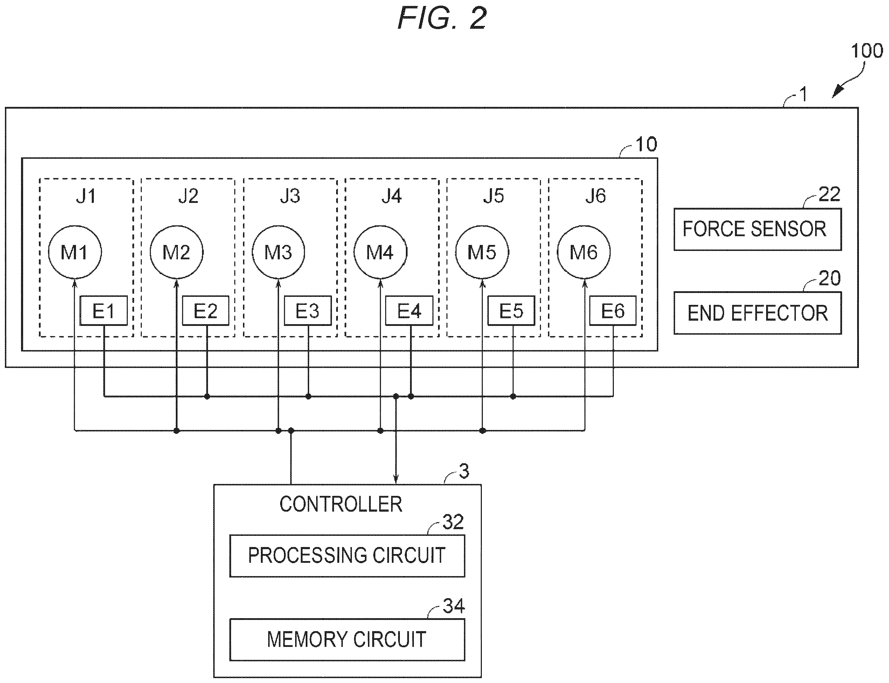

FIG. 1 is a perspective view showing the example of a robot system 100 to which the display system and the teaching system according to the present disclosure are applied. FIG. 2 is a block diagram for explanation of a basic configuration of the robot system 100 shown in FIG. 1. Note that, in FIG. 1 and the respective drawings to be described later, an x-axis, a y-axis, and a z-axis are set as three axes orthogonal to one another.

The robot system 100 shown in FIG. 1 includes a robot 2 and a controller 3.

### 1.1. Robot

The robot 2 includes e.g., a robot arm 10 having a plurality of arms and a plurality of joints J1 to J6, a base 11 supporting the robot arm 10, an end effector 20, and a force sensor 22.

The robot arm 10 is e.g., a manipulator having seven links mutually coupled by the six joints J1 to J6 and moving at six degrees of freedom. In the example shown in FIG. 1, the robot arm 10 includes the six joints J1 to J6 respectively as rotating joints. Note that the number of joints of the robot arm 10 is not particularly limited, but may be less than six, seven, or more. The base 11 supports the link closest to the base 11 side of the robot arm 10 via the joint J1.

The end effector 20 is a tool for various kinds of work of e.g., screwing, gripping, grinding, etc. The end effector 20 includes e.g., a screw driver, a gripper, and a grinder. The end effector 20 is attached to a mechanical interface at the distal end of the robot arm 10 via the force sensor 22. The robot arm 10 is controlled to drive by the controller 3 and determines a position and an attitude of the end effector 20.

Note that, in this specification, with reference to the robot arm 10, the base 11 side is referred to as "proximal end side" and the end effector 20 side is referred to as "distal end side".

The force sensor 22 detects an external force acting on a tool center point (TCP) as a reference for the position of the end effector 20 via e.g., the end effector 20. When subjected to an external force, the force sensor 22 outputs a signal corresponding to the external force to the controller 3. Thereby, the controller 3 detects forces along three detection axes and torque around the three detection axes acting on the TCP as external forces. The three detection axes form e.g., a world coordinate system defined by the x-axis, the y-axis, and the z-axis.

As shown in FIG. 2, the plurality of joints J1 to J6 have a plurality of motors M1 to M6 and a plurality of encoders E1 to E6. The motors M1 to M6 are respectively driven under control by the controller 3 and drive the joints J1 to J6, respectively. The encoders E1 to E6 detect and output rotation angles of the motors M1 to M6 to the controller 3.

The joint J1 is placed at the most proximal end side of the joints J1 to J6 and has the motor M1 and the encoder E1. The rotation axis of the joint J1 is along the z-axis.

The joint J2 is placed at the distal end side of the joint J1 and has the motor M2 and the encoder E2. The rotation axis of the joint J2 is along the xy-plane.

The joint J3 is placed at the distal end side of the joint J2 and has the motor M3 and the encoder E3. The rotation axis of the joint J3 is along the xy-plane.

The joint J4 is placed at the distal end side of the joint J3 and has the motor M4 and the encoder E4. The rotation axis of the joint J4 is orthogonal to the rotation axis of the joint J3.

The joint J5 is placed at the distal end side of the joint J4 and has the motor M5 and the encoder E5. The rotation axis of the joint J5 is orthogonal to the rotation axis of the joint J4.

The joint J6 is placed at the most distal end side of the joints J1 to J6 and has the motor M6 and the encoder E6. The rotation axis of the joint J6 is orthogonal to the rotation axis of the joint J5.

Note that the robot arm 10 shown in FIG. 1 is a vertical articulated robot arm, however, not limited to that. A horizontal articulated robot arm may be employed.

### 1.2. Controller

The controller 3 includes a processing circuit 32 and a memory circuit 34 forming a computer system. The processing circuit 32 realizes the respective functions by executing e.g., a control program stored in the memory circuit 34. As a circuit forming at least a part of the processing circuit 32, various logical operation circuits e.g., a central processing unit (CPU), a digital signal processor (DSP), a programmable logic device (PLD), an application specific integrated circuit (ASIC), etc. can be employed.

The memory circuit 34 is a computer-readable memory medium and stores the control program and various kinds of data necessary for the operation of the robot system 100. As the memory circuit 34, e.g., a semiconductor memory can be employed. The processing circuit 32 and the memory circuit 34 may be formed by integrated hardware or formed by a plurality of separate pieces of hardware. Part or all of the component elements of the controller 3 may be placed inside of the housing of the robot 2.

The controller 3 drives the motors M1 to M6 according to the control program. The controller 3 detects the rotation angles acquired by the respective encoders E1 to E6 and controls driving of the motors M1 to M6 based on the detection results.

### 2. Configuration of Teaching System

Next, configurations of the display system and the teaching system according to an embodiment will be explained.

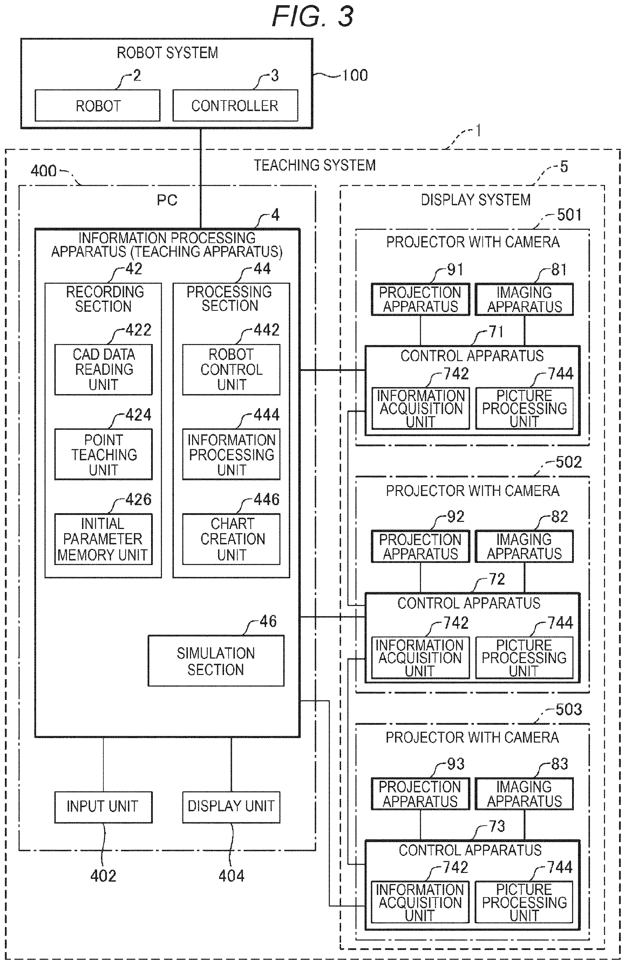

FIG. 3 is a block diagram showing the configuration and functions of a teaching system 1 according to the embodiment.

The teaching system 1 shown in FIG. 3 includes an information processing apparatus 4 (teaching apparatus), a display system 5, an input unit 402, and a display unit 404.

The information processing apparatus 4 has a function of teaching a predetermined motion to the robot 2 and simulating the taught motion. The display system 5 projects information including characters and figures on the outer surface of the robot 2. The projected information may include e.g., information for supporting teaching of the robot 2. The input unit 402 receives an input operation by a teaching worker. The display unit 404 displays arbitrary information separately from the information projected on the outer surface of the robot 2 by the display system 5.

The teaching worker may directly visually recognize the robot arm 10 being reproducing the taught motion while visually recognizing the information projected on the outer surface of the robot 2 by the display system 5. That is, the teaching worker may acquire information on teaching by visually recognizing the outer surface of the robot 2, for example, without visually recognizing a display screen provided in a position apart from the robot 2 such as the display unit 404 or when the number of times of visual recognition is reduced. Thereby, the teaching worker may perceive both the real motion of the robot arm 10 and the information on teaching while minimizing the movement of the point of view. As a result, efficiency of the teaching work may be increased.

The input unit 402 receives the input operation by the teaching worker. The input unit 402 includes e.g., a keyboard, a mouse, a touch panel, and a teaching pendant. The display unit 404 displays arbitrary information separately from the information projected on the outer surface of the robot 2 by the display system 5. The display unit 404 includes e.g., a liquid crystal display device.

The respective functions of the information processing apparatus 4 are realized by hardware including e.g., a processor, a memory, and an external interface. The processor includes e.g., a CPU (Central Processing Unit). The memory includes one or both semiconductor memories of a volatile memory such as a RAM (Random Access Memory), a nonvolatile memory such as a ROM (Read Only Memory), an EEPROM (Electrically Erasable Programmable Read-Only Memory), or a PROM (Programmable ROM). The external interface includes e.g., a digital input/output port such as a USB (Universal Serial Bus) and an Ethernet (registered trademark) port. The respective functions are realized by the processor executing the programs stored in the memory.

Note that, as the processor, a programmable logic device such as an FPGA (field-programmable gate array) may be used in place of the CPU or in addition to the CPU.

For example, the hardware includes not only a personal computer (PC 400) shown in FIG. 3 but also a tablet terminal and a smartphone.

The display system 5 is a system projecting and displaying information on the robot 2 while the robot 2 is moving. The display system 5 includes three control apparatuses 71, 72, 73, three imaging apparatuses 81, 82, 83, and three projection apparatuses 91, 92, 93.

The control apparatuses 71 to 73 have functions of outputting picture signals to the projection apparatuses 91 to 93 based on an information processing result output from the information processing apparatus 4 and images captured by the imaging apparatuses 81 to 83, respectively.

The imaging apparatuses 81 to 83 have functions of respectively imaging the robot 2 and outputting the images to the information processing apparatus 4.

The projection apparatuses 91 to 93 have functions of projecting information on the robot 2 based on the picture signals output from the control apparatuses 71 to 73, respectively.

The respective functions of the control apparatuses 71 to 73, the imaging apparatuses 81 to 83, and the projection apparatuses 91 to 93 are realized by e.g., projectors with camera 501 to 503 shown in FIG. 3. The respective functions of the control apparatuses 71 to 73 are realized by hardware including e.g., processors, memories, and external interfaces.

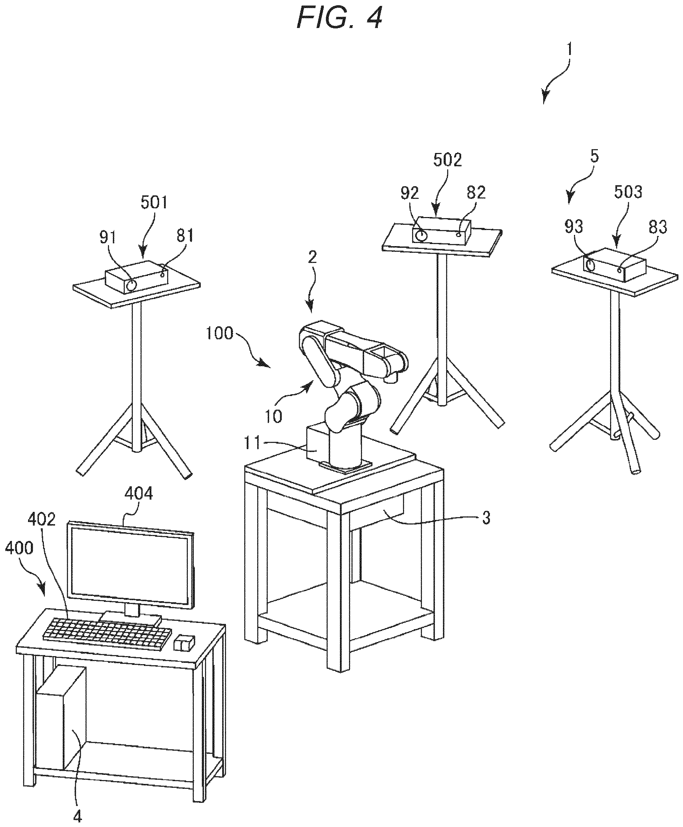

FIG. 4 is a perspective view showing a placement example of the teaching system 1 and the robot system 100 shown in FIG. 3.

As shown in FIG. 4, the imaging apparatuses 81 to 83 and the projection apparatuses 91 to 93 are placed to surround the robot system 100. Further, in FIG. 4, the PC 400 is placed near the robot system 100, but may be placed in a remote location.

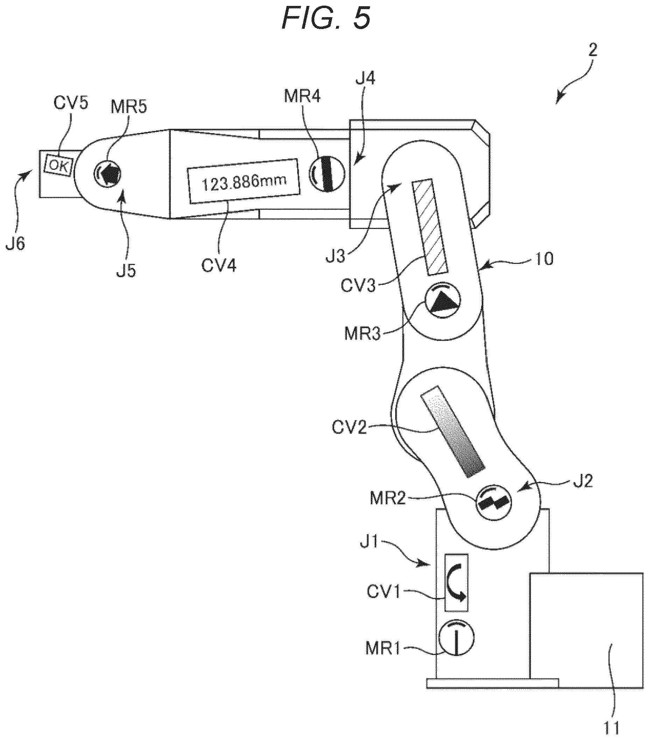

FIG. 5 is a side view showing examples of markers MR1 to MR5 and canvases CV1 to CV5 attached to the outer surface of the robot 2.

As shown in FIG. 5, the markers MR1 to MR5 are attached near the joints J1 to J5 on the outer surface of the robot 2. Though not shown in FIG. 5, a marker MR6 may be attached near the joint J6. The markers MR1 to MR6 respectively have unique patterns. These markers MR1 to MR6 are imaged by the imaging apparatuses 81 to 83, and thereby, the respective pivot angles of the joints J1 to J6, i.e., an attitude of the robot 2 may be detected.

Further, as shown in FIG. 5, on the outer surface of the robot 2, the canvas CV1 is set to correspond to the marker MR1, the canvas CV2 is set to correspond to the marker MR2, the canvas CV3 is set to correspond to the marker MR3, the canvas CV4 is set to correspond to the marker MR4, and the canvas CV5 is set to correspond to the marker MR5. Though not shown in FIG. 5, a canvas CV6 may be set to correspond to the marker MR6. On these canvases CV1 to CV6, information is projected from the projection apparatuses 91 to 93. Thereby, the teaching worker may easily associate the information projected on the canvases CV1 to CV6 with the corresponding joints J1 to J6 by visually recognizing the information. The canvases CV1 to CV6 may be set on non-flat surfaces such as curved surfaces or concave-convex surfaces of the outer surface of the robot 2, but preferably set on flat surfaces. Thereby, distortion of the projected information may be suppressed.

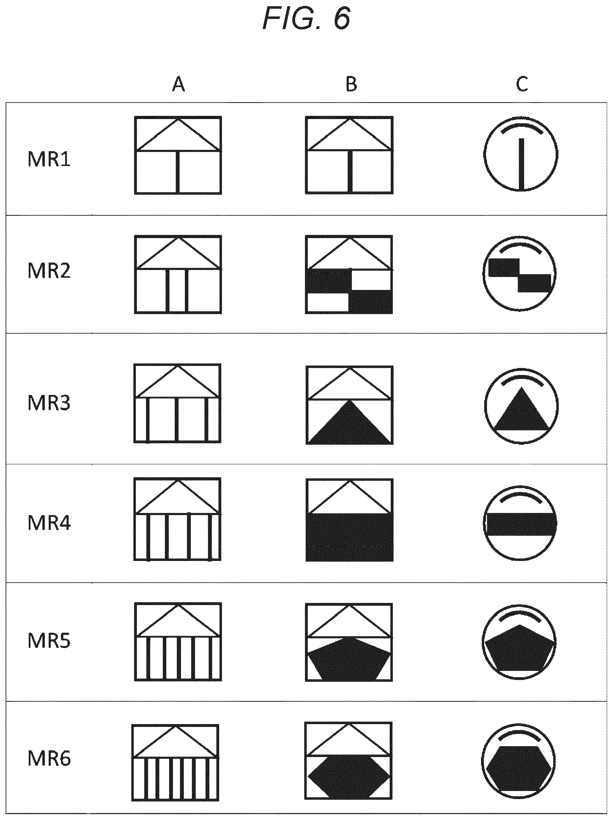

FIG. 6 is a table showing examples of the markers MR1 to MR6. FIG. 6 exemplifies respective three types of type A, type B, and type C of the markers MR1 to MR6.

Type A is of a marker of a combination of a triangle and bars. The triangles indicate directions of the canvases CV1 to CV6 by positions of blunt angles. Further, the numbers of bars indicate the positions of the joints. For example, when the number of bars is one, the marker shows the joint J1. In the display system 5, the markers MR1 to MR6 are identified by an image recognition technique, and thereby, the joints J1 to J6 can be identified.

Type B is of a marker of a combination of an open triangle and a solid polygon. The open triangles indicate directions of the canvases CV1 to CV6. The solid polygons indicate the positions of the joints by the shapes thereof.

Type C is of a marker of a combination of an arc and a polygon. The arcs indicate directions of the canvases CV1 to CV6 by the positions thereof. The polygons have the same meanings as those of Type B.

As described above, the markers MR1 to MR6 have functions identifying the joints J1 to J6 and functions identifying the directions of the canvases CV1 to CV6. The markers MR1 to MR6 having those functions are imaged by the imaging apparatuses 81 to 83, and thereby, the positions and the pivot states of the joints J1 to J6 may be acquired by the control apparatuses 71 to 73.

Note that the markers MR1 to MR6 are not limited to those illustrated as long as the markers can be distinguished from one another by the image recognition technique. For example, the markers MR1 to MR6 may be one-dimensional barcodes or two-dimensional barcodes.

### 2.1. Information Processing Apparatus (Teaching Apparatus)

The information processing apparatus 4 has the function of teaching a predetermined motion to the robot 2 and simulating the taught motion. The information processing apparatus 4 has a recording section 42, a processing section 44, and a simulation section 46.

### 2.1.1. Recording Section

The recording section 42 has a function of acquiring and recording information necessary for a simulation and a function of outputting the recorded information as teaching information to the processing section 44. The recording section 42 has a CAD data reading unit 422, a point teaching unit 424, and an initial parameter memory unit 426.

The CAD data reading unit 422 reads CAD (Computer Aided Design) data of the robot 2, peripheral devices therefor, etc. The read CAD data is provided for information processing in the processing section 44 and simulations in the simulation section 46.

The point teaching unit 424 reads point data necessary for teaching of the robot 2. The point data is data for teaching the motion of the robot 2 by e.g., designation of the position and the attitude of the end effector 20 or the like. The point data may be read via an external memory device (not shown) or a network or input via the input unit 402.

The initial parameter memory unit 426 stores an initial value of a motion parameter. The motion parameter is an initial value of a parameter necessary for the simulation section 46 to perform a simulation of the motion of the robot 2. In addition, the initial parameter memory unit 426 may store types of information to be displayed in the display system 5 in advance.

### 2.1.2. Processing Section

The processing section 44 has a function of outputting a control signal for moving the robot 2 and a control signal for controlling the operation of the display system 5 based on the teaching information output from the recording section 42 and a result of the simulation output from the simulation section 46. The processing section 44 has a robot control unit 442, an information processing unit 444, and a chart creation unit 446.

The robot control unit 442 outputs a control signal relating to a position and an attitude of the robot arm 10 of the robot 2 to the controller 3. The controller 3 controls driving of the robot arm 10 based on the input control signal. Further, the robot control unit 442 acquires motion information of the robot 2, e.g., a velocity and an acceleration of the end effector 20, angular velocities and angular accelerations of the joints J1 to J6, torque of the joints J1 to J6, an elapsed time from a reference time, etc. from the controller 3.

The information processing unit 444 creates information to be displayed in the display system 5 based on the result of the simulation by the simulation section 46.

The chart creation unit 446 creates a chart based on the result of the simulation by the simulation section 46 or the information output from the information processing unit 444. Note that, in this specification, "chart" refers to a representation of numerical value data by figures. The chart includes a graph. The created chart is output to the display system 5.

The simulation section 46 simulates the motion of the robot 2 and an interference between the robot 2 and a peripheral device or the like. Then, the simulation section 46 outputs a result of the simulation to the information processing unit 444 or the chart creation unit 446.

Note that, in the following description, a result of the information processing by the information processing apparatus 4 is referred to as "information processing result".

Part of the functions of the information processing apparatus 4 may be realized by the projectors with camera 501 to 503 or other external devices.

### 2.2. Display System

As shown in FIG. 3, the display system 5 includes the three control apparatuses 71, 72, 73, the three imaging apparatuses 81, 82, 83, and the three projection apparatuses 91, 92, 93.

### 2.2.1. Control Apparatuses

The control apparatuses 71 to 73 have functions of outputting picture signals to the projection apparatuses 91 to 93, respectively. The control apparatuses 71 to 73 respectively have information acquisition units 742 and picture processing units 744.

The information acquisition units 742 have functions of acquiring the information processing result from the information processing apparatus 4 and acquiring images from the imaging apparatuses 81 to 83. The information processing result acquired from the information processing apparatus 4 may be e.g., characters, figures containing charts, etc. Further, the information acquisition units 742 perform image recognition processing on the acquired images and detect positions, sizes, shapes, directions, etc. of the markers MR1 to MR6. The information acquisition units 742 have information of relative positions, sizes, etc. of the canvases CV1 to CV6 to the markers MR1 to MR6 in advance. The information acquisition units 742 specify the positions, the sizes, etc. of the canvases CV1 to CV6 in a space based on the information and the detected markers MR1 to MR6. Then, the information acquisition units 742 convert the positions and the sizes into positions and sizes of the canvases CV1 to CV6 in projectable ranges of the projection apparatuses 91 to 93.

Note that the display system 5 according to the embodiment includes the plurality of imaging apparatuses 81 to 83. When the same markers appear in a plurality of images acquired from the imaging apparatuses 81 to 83, the information acquisition units 742 select one images and detect the markers MR1 to MR6. In this case, it is preferable to select the images in which the respective markers are captured from sides closest to the front side. Thereby, detection accuracy of the markers is increased and, in the picture processing units 744, processing of pictures according to the positions and the sizes of the canvases CV1 to CV6 may be performed more accurately.

The picture processing units 744 have functions of creating and outputting picture signals to be projected by the projection apparatuses 91 to 93 based on the acquisition results by the information acquisition units 742. Specifically, the units create the picture signals formed by processing of the pictures representing the information processing result output from the information processing apparatus 4 according to the positions, the sizes, etc. of the canvases CV1 to CV6. The picture signals are output to the projection apparatuses 91 to 93, and thereby, individual information may be respectively displayed in the canvases CV1 to CV6. Examples of the processing include, e.g., enlargement, reduction, deformation, rotation, etc. of the pictures according to the positions and the sizes of the canvases CV1 to CV6. Note that, when all of the markers MR1 to MR6 appear in a single image, the image may be selected, however, because of the structure of the robot arm 10, the markers MR1 to MR6 separately appear in a plurality of images. Therefore, the information acquisition units 742 detect the markers MR1 to MR6 from the plurality of images. Thereby, the images from which the markers MR1 to MR6 are properly detectable may be acquired regardless of the attitude of the robot 2.

Further, the display system 5 according to the embodiment includes the plurality of projection apparatuses 91 to 93. The picture processing units 744 store relative positions of the projection apparatuses 91 to 93 to the robot 2 in advance. Accordingly, the picture processing units 744 select ones from the plurality of projection apparatuses 91 to 93 and control the apparatuses to project the information based on the detection results of the markers MR1 to MR6 by the information acquisition units 742. In this case, it is preferable to select the projection apparatuses 91 to 93 that may project the information from the sides closest to the front side onto the canvases CV1 to CV6. Thereby, image quality of the projected information is increased and visibility by the teaching worker may be increased. Note that some of the projection apparatuses 91 to 93 may be selected to project the information.

The control apparatuses 71 to 73 are configured to communicate with one another. Thereby, the control apparatuses 71 to 73 may cooperatively control the projection apparatuses 91 to 93. As a result, the control apparatuses 71 to 73 are configured to perform control to select one, two, or more of the projection apparatuses 91 to 93 based on e.g., the position relationship between the robot 2 and the projection apparatuses 91 to 93 and project information on the robot 2. Thereby, information may be projected from the appropriate projection apparatuses 91 to 93 according to the directions of the canvases CV1 to CV6, and highly visible information may be projected. For the technique of cooperatively controlling the plurality of projection apparatuses, e.g., an existing multi-projection technique may be used.

Further, the display system 5 and the information processing apparatus 4 are configured to communicate with each other. Thereby, the information processing result output from the information processing apparatus 4 is respectively input to the control apparatuses 71 to 73.

Note that the control apparatuses 71 to 73 may be aggregated to a single apparatus or the functions of the control apparatuses 71 to 73 may be realized by the PC 400. Or, the control apparatuses 71 to 73 may be configured not to communicate with each other.

### 2.2.2. Imaging Apparatuses

The imaging apparatuses 81 to 83 have functions of imaging the robot 2 and outputting images to the control apparatuses 71 to 73, respectively. The respective imageable ranges (angles of view) of the imaging apparatuses 81 to 83 may be set to parts of the robot 2, however, are preferably set to the entire of the robot 2. Thereby, it is possible that a single imaging apparatus may image all of the markers MR1 to MR6 depending on the attitude of the robot 2. As a result, the number of necessary imaging apparatuses may be reduced.

The display system 5 includes the plurality of imaging apparatuses 81 to 83, and thereby, may image the robot 2 from multiple directions. Thereby, it is highly probable that one or some of the imaging apparatuses 81 to 83 may image the markers MR1 to MR6. Particularly, when the number of imaging apparatuses is three or more, the probability may be especially increased. In the embodiment, the case where the number of imaging apparatuses is three is explained as an example.

The imaging apparatuses 81 to 83 include e.g., CCD (Charge Coupled Device) cameras, CMOS (Complementary Metal Oxide Semiconductor) cameras, etc.

### 2.2.3. Projection Apparatuses

The projection apparatuses 91 to 93 respectively have functions of projecting information on the robot 2. The respective projectable ranges (angles of view) of the projection apparatuses 91 to 93 may be set to parts of the robot 2, however, are preferably set to the entire of the robot 2. Thereby, it is possible that a single projection apparatus may project on all of the canvases CV1 to CV6 depending on the attitude of the robot 2. As a result, the number of necessary projection apparatuses may be reduced.

The display system 5 includes the plurality of projection apparatuses 91 to 93, and thereby, may display information on the robot 2 from multiple directions. Thereby, it is highly probable that one or some of the projection apparatuses 91 to 93 may project information on the canvases CV1 to CV6. Particularly, when the number of projection apparatuses is three or more, the probability may be especially increased. In the embodiment, the case where the number of projection apparatuses is three is explained as an example.

The information projected by the projection apparatuses 91 to 93 represents the taught motion of the robot 2 by e.g., characters including numerical values and texts and figures including patterns and charts. The information is projected, and thereby, for example, the teaching worker teaching the motion of the robot 2 may perceive both the real motion of the robot arm 10 and the information on teaching while minimizing the movement of the point of view. Thereby, efficiency of the teaching work may be increased.

For example, when a monitor or the like is placed in the robot arm 10 or the base 11 of the robot 2 to display information, the monitor and the robot arm 10 may interfere. Further, a blind spot is produced due to the monitor and workability of teaching may be lower. On the other hand, according to the display system 5, the problem may be solved. Further, it is unnecessary to place the monitor, and a space for the teaching worker to work may be easily secured and increase in weight and wire length of the robot 2 may be suppressed. Furthermore, the teaching worker may visually recognize the real motion of the robot arm 10 while visually recognizing information on teaching without using a teaching pendant with a monitor, and there is an advantage that the teaching worker may freely use both hands during the visual recognition.

Each of the projection apparatuses 91 to 93 has a projection unit (not shown). The projection unit has e.g., a light source, a light modulation device, a projection optical system, etc. The light modulation device includes e.g., a liquid crystal light valve. The liquid crystal light valve has a rectangular pixel area of a plurality of pixels arranged in a matrix form. In the liquid crystal light valve, light transmissivity with respect to each pixel may be changed based on the picture signal. The light output from the light source is transmitted through the pixel area and modulated, and a picture according to the picture signal is projected on the outer surface of the robot 2. When the liquid crystal light valves are divided for each of the three primary colors of light, a color picture may be projected according to color information contained in the picture signal.

In the placement example shown in FIG. 4, the imaging apparatus 81 and projection apparatus 91, the imaging apparatus 82 and projection apparatus 92, and the imaging apparatus 83 and projection apparatus 93 are respectively placed close to each other. Thereby, for example, if the imaging apparatus 81 can image the marker MR1, a high probability that information can be projected on the canvas CV1 by the projection apparatus 91 may be determined. As described above, sets of one imaging apparatus and one projection apparatus are placed, and thereby, the load of picture processing in the projection apparatuses 91 to 93 may be reduced. Note that the placement of the imaging apparatuses 81 to 83 and the projection apparatuses 91 to 93 is not limited to the placement shown in FIG. 4. For example, the imaging apparatus 81 and the projection apparatus 91 may be placed apart from each other. Or, the numbers of the imaging apparatuses and the projection apparatuses may be different from each other.

The above described display system 5 is preferably configured to operate in real time. That is, when the attitude of the robot 2 changes, the change of the attitude may be detected from the images acquired by the imaging apparatuses 81 to 83. Thereby, the information displayed by the display system 5 may be changed according to the changes of the positions and the sizes of the canvases CV1 to CV6. As a result, the display system 5 may change the displayed information in real time to follow the changes in attitude of the robot 2.

Further, the display system 5 may be used for other use than teaching of the motion to the robot 2, e.g., use to check the motion of the robot 2 to which the motion is taught.

### 3. Teaching Method

Next, an example of a teaching method using the teaching system according to the embodiment will be explained.

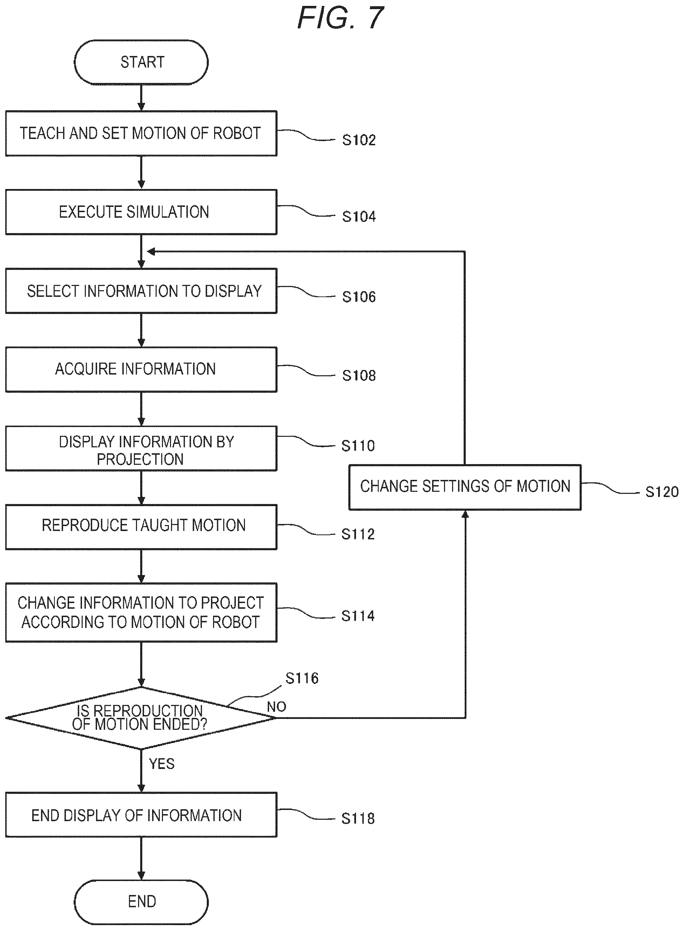

FIG. 7 is a flowchart for explanation of the example of the teaching method using the teaching system according to the embodiment.

At step S102, first, as shown in FIG. 4, the projectors with camera 501 to 503 are placed around the robot system 100.

Then, the relative positions of the projectors with camera 501 to 503 to the robot 2 are registered in the control apparatuses 71 to 73. For the registrations, e.g., a marker for calibration placed on the base 11 of the robot 2 is used. The marker is imaged by the imaging apparatuses 81 to 83 and the position, the size, etc. of the maker are detected. Thereby, the relative positions of the imaging apparatuses 81 to 83 to the robot 2 may be registered. Further, the relative positions of the projection apparatuses 91 to 93 to the robot 2 may be registered based on the relative positions of the projection apparatuses 91 to 93 to the imaging apparatuses 81 to 83.

Then, teaching of the motion of the robot 2 is performed. For example, the point data is read by the information processing apparatus 4 and a path of the end effector 20 is created. Further, the motion of the robot 2 is set. For example, the information processing apparatus 4 is controlled to read the motion parameter and create a trajectory.

At step S104, a simulation to move the robot arm 10 in the created trajectory is performed by the simulation section 46. Then, the information processing unit 444 creates information to be displayed by the display system 5 based on the result of the simulation.

At step S106, for the teaching worker to select the type of information to be projected on the robot 2, the types of information are displayed on the display unit 404. Then, input is received by the input unit 402, and thereby, the selected information is recorded.

At step S108, the information processing result from the information processing apparatus 4 is acquired by the control apparatuses 71 to 73 and the images are acquired from the imaging apparatuses 81 to 83. Further, the markers MR1 to MR6 are detected. Then, the picture signals to be projected by the projection apparatuses 91 to 93 are created.

At step S110, the information is projected on the robot 2 by the display system 5. Thereby, the information is displayed on the robot 2.

At step S112, the taught motion is reproduced by the robot control unit 442. Thereby, the robot 2 really moves.

At step S114, the information projected on the robot arm 10 is changed according to the motion of the robot 2 by the display system 5. Thereby, the projected information may be changed in real time. Specifically, characters, figures, colors, blinking, etc. representing the information are changed in real time according to the motion of the robot 2. Thereby, the teaching worker may easily intuitively perceive meaning of the information.

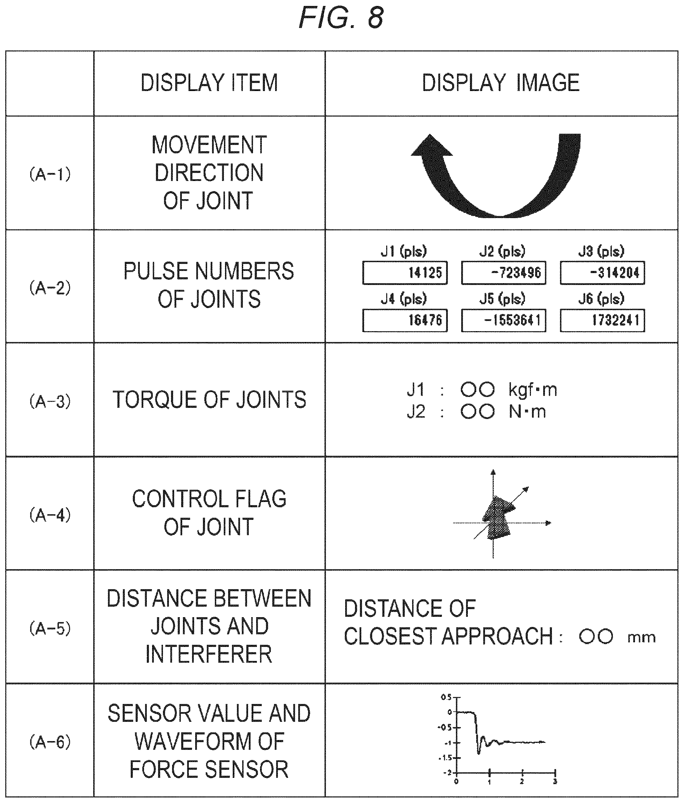

FIG. 8 is a table showing examples of types of information displayed by the display system 5 according to the embodiment.

As shown in FIG. 8, for example, the information displayed by the display system 5 includes:
(A-1) movement directions of the joints J1 to J6;
(A-2) pulse numbers of the joints J1 to J6;
(A-3) torque of the joints J1 to J6;
(A-4) control flags of the joints J1 to J6;
(A-5) distances between the joints J1 to J6 and interferers; and
(A-6) a sensor value and a waveform of the force sensor 22.
Also, visual images of displayed information are shown in FIG. 8.

As an example of the information (A-1), in the canvas CV1 shown in FIG. 5, the movement direction of the joint J1 is displayed by an arrow. The canvas CV1 is set near the joint J1. Accordingly, the teaching worker may visually recognize the movement direction of the joint J1 displayed in the canvas CV1 and the real motion of the joint J1 in the same field of view, and thereby, a status of teaching may be intuitively checked with less burden.

For example, as shown in FIG. 8, the information (A-2) and the information (A-3) are displayed by numerical values and texts. The information containing characters is displayed, and thereby, information with less misunderstandings may be transmitted to the teaching worker. Note that the control flags include e.g., values indicating whether pivot angles from reference angles of the joints J1 to J6 are equal to or larger than thresholds, values representing a particular attitude of the robot arm 10 by terms including a hand (Hand), an elbow (Elbow), and a wrist (Wrist), etc.

As other examples of the information (A-2) and the information (A-3), information containing characters including numerical values and texts is displayed in the canvases CV4, CV5 shown in FIG. 5.

For example, as shown in FIG. 8, the information (A-4) is displayed by an arrow. The arrow intuitively indicates e.g., a direction of an object (interferer) that may interfere in a position closest to the corresponding joint utilizing the characteristics of the figure. Or, a distance to the interferer may be displayed by a numerical value. In this case, when the distance to the interferer is particularly short, the color of the displayed information may be changed. That is, the color displayed by the display system 5 may be a single color, but preferably be multiple colors. Thereby, the information changes may appeal to the sense of vision of the teaching worker and, for example, changes of situations including e.g., the degree of urgency may be transmitted in an easy-to-understand manner.

For example, as shown in FIG. 8, the information (A-5) is displayed by numerical values and texts. As another example of the information (A-5), figures showing the distances to the interferers etc. by changes of colors and patterns are displayed in the canvases CV2, CV3 shown in FIG. 5.

For example, as shown in FIG. 8, the information (A-6) is displayed by a graph (chart) indicating a sensor value and a waveform of the force sensor 22 or the like.

In addition to the above described information, though not shown, examples of the information displayed by the display system 5 include:
(B-1) a current position of the robot arm 10;
(B-2) a real value or an assumed value in a simulation of an acceleration or deceleration of the robot arm 10;
(B-3) a distance to a destination of the robot arm 10;
(B-4) a cumulative movement distance of the robot arm 10;
(B-5) a distance of closest approach to an interferer in a TCP coordinate system;
(B-6) a motion time of the robot 2 or a motion time in a simulation;
(C-1) a status of I/O (input/output) to and from the robot 2;
(C-2) a status of a motion command to the robot 2; and
(C-3) a value of a variable arbitrarily defined by the teaching worker.
The information is displayed by e.g., characters including numerical values and texts, figures including charts, or the like.

As described above, the information projected on the robot 2 and the real motion of the robot 2 may be compared, and thereby, for example, checking as to whether the taught motion contains an unnecessary motion, perception of a tendency of a real motion of the robot with the motion, prior perception of a risk such as an overload, etc. are easier. Accordingly, teaching contents may be efficiently improved and failures may be early predicted.

The displayed contents may be fixed, but preferably changed in real time according to the progress of the taught motion. Thereby, the teaching worker may perceive the relationship between the taught motion and the motion of the robot 2 more properly.

Particularly, all of the information (A-1) to (A-6) is information unique to the respective joints. Accordingly, the information unique to the joint is projected on the canvas corresponding to the joint, and thereby, the teaching worker may perceive the relationship between the information and the joint more intuitively. As a result, the teaching worker may improve the teaching contents and predict failures more efficiently.

At step S116, whether the reproduction of the taught motion is ended is determined. For example, in a case where, as a result of the comparison between the information projected on the robot 2 and the real motion of the robot 2, failures or the like are not recognized and a determination that improvements of the teaching contents are unnecessary is made or the like, the reproduction of the motion is ended. When the reproduction of the motion is ended, that is, the result of the determination is YES, the teaching worker inputs an operation to instruct to end from the input unit 402. Thereby, the processing goes to step S118. At step S118, the information display by the display system 5 is ended. Thereby, the taught motion may be regarded as being good.

On the other hand, when the reproduction of the motion is not ended, that is, the result of the determination is NO, the processing goes to step S120. For example, in a case where failures are recognized in the real motion of the robot 2 in the reproduction of the motion or the like, it is necessary to continue checking of the motion without ending the reproduction of the motion. Accordingly, at step S120, the settings of the motion are changed. For example, the information processing apparatus 4 is controlled to read the motion parameter different from the initial value of the motion parameter read at step S102, and a new trajectory is created. Then, the processing goes to step S106. At step S106, information to be displayed is selected again with respect to the new trajectory.

In the above described manner, changing of the motion parameter is repeated until any failures are no longer recognized in the motion of the robot 2. Thereby, the teaching of the robot 2 is completed.

### 4. Effects exerted by Embodiment

As described above, the display system 5 according to the embodiment is a system displaying information on the robot arm 10 when the robot arm 10 including the joints J1 to J6 is moving. The display system 5 includes the plurality of imaging apparatuses 81 to 83, the plurality of projection apparatuses 91 to 93, and the control apparatuses 71 to 73. The imaging apparatuses 81 to 83 image the markers MR1 to MR6 provided on the robot arm 10. The projection apparatuses 91 to 93 project the information on the robot arm 10. The control apparatuses 71 to 73 project the information to follow the moving robot arm 10 based on the images of the markers MR1 to MR6 captured by the imaging apparatuses 81 to 83.

According to the display system 5, the worker may efficiently visually recognize the information on the motion of the robot arm 10 while visually recognizing the real motion of the robot arm 10. Thereby, when the display system 5 is used for the teaching of the motion of the robot 2, the teaching worker may perceive both the real motion of the robot arm 10 and the information on the teaching while minimizing the movement of the point of view. Thereby, the efficiency of the teaching work may be increased.

Further, it is not necessary to place a monitor or the like in the robot 2, and there are advantages that a space for the worker to work may be easily secured and the increase in weight and wire length of the robot 2 may be suppressed. Furthermore, the teaching worker may visually recognize the real motion of the robot arm 10 while visually recognizing the information on teaching without using a teaching pendant with a monitor, and there is an advantage that the teaching worker may freely use both hands during the visual recognition.

In the display system 5 according to the embodiment, the information projected by the projection apparatuses 91 to 93 includes the movement directions of the joints J1 to J6, the pulse numbers of the joints J1 to J6, the torque of the joints J1 to J6, the control flags of the joints J1 to J6, or the distances between the joints J1 to J6 and interferers.

According to the configuration, the worker may visually recognize the movement direction of the joint J1 displayed in the canvas CV1 and the real motion of the joint J1 in the same field of view. Accordingly, when the display system 5 is used for the teaching of the motion of the robot 2, the status of teaching may be intuitively checked with less burden.

In the display system 5 according to the embodiment, the robot arm 10 includes the plurality of joints J1 to J6. The projection apparatuses 91 to 93 project information on the canvases CV1 to CV6 as a plurality of areas corresponding to the plurality of joints J1 to J6.

According to the configuration, the joints J1 to J6 correspond to the canvases CV1 to CV6, and thereby, the worker may easily associate the information projected on the respective canvases with the corresponding joints.

In the display system 5 according to the embodiment, the projection apparatuses 91 to 93 project the information unique to the joints J1 to J6 on the canvases CV1 to CV6 as the areas corresponding to the joints J1 to J6.

According to the configuration, the information unique to the joints J1 to J6 is projected on the canvases CV1 to CV6 corresponding to the joints J1 to J6, and thereby, the worker may perceive the relationship between the information and the joints J1 to J6 more intuitively.

In the display system 5 according to the embodiment, the information projected by the projection apparatuses 91 to 93 contains characters or figures.

When the projected information contains characters, information with less misunderstandings may be transmitted to the worker. Or, when the projected information contains figures, the information may be intuitively transmitted to the worker utilizing the characteristics of the figures.

In the display system 5 according to the embodiment, the information projected by the projection apparatuses 91 to 93 contains a plurality of colors.

According to the configuration, the colors of the displayed information may be changed, and thereby, the information changes may appeal to the sense of vision of the worker and, for example, changes of situations including e.g., the degree of urgency may be transmitted in an easy-to-understand manner.

The teaching system 1 according to the embodiment includes the display system 5 and the information processing apparatus 4 (teaching apparatus). The information processing apparatus 4 teaches the motion of the robot 2 including the robot arm 10.

According to the teaching system 1, the teaching worker may perceive both the real motion of the robot arm 10 and the information on the teaching while minimizing the movement of the point of view. Thereby, the efficiency of the teaching work may be increased.

Further, it is not necessary to place a monitor or the like in the robot 2, and there are advantages that a space for the teaching worker to perform teaching work may be easily secured and the increase in weight and wire length of the robot 2 may be suppressed. Furthermore, the teaching worker may visually recognize the real motion of the robot arm 10 while visually recognizing the information on teaching without using a teaching pendant with a monitor, and there is an advantage that the teaching worker may freely use both hands during the visual recognition.

As above, the display system and the teaching system according to the present disclosure are explained based on the illustrated embodiment, however, the present disclosure is not limited to that.

For example, in the display system and the teaching system according to the present disclosure, the respective units of the above described embodiment may be respectively replaced by any configurations having the same functions and any configurations may be added to the embodiment.

## Claims

1. A display system displaying information on a robot arm when the robot arm having a joint is moving, comprising:
a plurality of imaging apparatuses imaging markers provided on the robot arm;
a plurality of projection apparatuses projecting the information on the robot arm; and
a control apparatus controlling operation of the projection apparatuses to project the information to follow the moving robot arm based on images of the markers captured by the imaging apparatuses.

2. The display system according to claim 1, wherein
the information includesa movement direction of the joint, a pulse numberof the joint, torque of the joint, a control flag of the joint, or a distances between the joint and an interferer.

3. The display system according to claim 1, wherein
the robot arm includes a plurality of the joints, and
the projection apparatuses project the information in a plurality of areas corresponding to the plurality of joints.

4. The display system according to claim 3, wherein
the projection apparatuses project the information unique to the joints in the areas corresponding to the joints.

5. The display system according to claim 1, wherein
the information contains a character or a figure.

6. The display system according to claim 1, wherein
the information contains a plurality of colors.

7. A teaching system comprising:
the display system according to claim 1; and
a teaching apparatus teaching a motion of a robot including the robot arm.
